# EUROPÄISCHE PATENTSCHRIFT

(11) **EP 0 340 718 B1**
(45) Veröffentlichungstag und Bekanntmachung des Hinweises auf die Patenterteilung: **20.07.1994**
(21) Anmeldenummer: 89107923.8
(22) Anmeldetag: 02.05.1989
(51) Int. Cl.: C07D 405/04, C07D 405/12

(54) **Chromanderivate**
Chroman derivatives
Dérivés de chromane

(30) Priorität: 06.05.1988 DE 3815504; 14.10.1988 DE 3835011
(43) Veröffentlichungstag der Anmeldung: 08.11.1989
(73) Patentinhaber: MERCK PATENT GmbH, D-64271 Darmstadt (DE)
(72) Erfinder: Gericke, Rolf, Dr., D-6104 Seeheim (DE); Baumgarth, Manfred, Dr., D-6100 Darmstadt (DE); Lues, Ingeborg, Dr., D-6100 Darmstadt (DE); Bergmann, Rolf, Dr., D-6101 Reichelsheim (DE); De Peyer, Jacques, Dr., D-6102 Pfungstadt (DE)

(56) Entgegenhaltungen:
- EP-A- 0 076 075
- EP-A- 0 107 423
- EP-A- 0 120 427
- EP-A- 0 273 262
- GB-A- 2 204 868
- JOURNAL OF MEDICINAL CHEMISTRY, Band 29, Nr. 11, 1986, Seiten 2194-2201
- JOURNAL OF THE CHEMICAL SOCIETY, PERKIN TRANSACTIONS I, Band 12, 1983, Seiten 2903-2912
- CHEMICAL ABSTRACTS, Band 84, Nr. 5, 2. Februar 1976, Seite 451, Spalte 1, Zusammenfassung-Nr. 30936m, Columbus, Ohio, USA
- JOURNAL OF MEDICINAL CHEMISTRY, Band 34, Nr. 10, 1991, Seiten 3074-3085

## Beschreibung

Die Erfindung betrifft neue Chromanderivate der Formel I 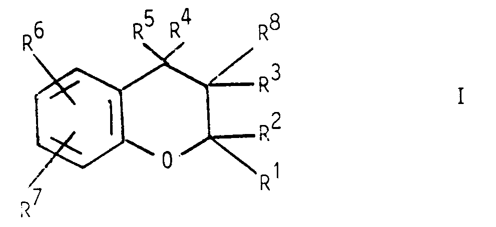
worin
- R¹ und R⁸: jeweils A,
- R²: H oder A,
- R¹ und R²: zusammen auch Alkylen mit 3-6 C-Atomen,
- R³: OH oder OAc,
- R⁴: H,
- R³ und R⁴: zusammen auch eine Bildung,
- R⁵: einen unsubstituierten oder ein- oder zweifach durch A, F, Cl, Br, J, OH, OA, OAc, NO₂, NH₂, AcNH, HOOC und/oder AOOC substituierten Pyridyl-oxy-, Pyridazinyl-oxy-, Pyrimidinyl-oxy-, Pyrazinyl-oxy-, Oxo-dihydro-pyridyl-oxy-, Oxo-dihydro-pyridazinyl-oxy-, Oxo-dihydro-pyrimidinyl-oxy- oder Oxo-dihydro-pyrazinyl-oxy-rest,
- R⁶ und R⁷: jeweils H, A, HO, AO, CHO, ACO,ACS, HOOC, AOOC, AO-CS,ACOO, A-CS-O, Hydroxy-alkylen, Mercapto-alkylen, NO₂, NH₂, NHA, NA₂, CN,F,Cl,Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylen, Nitro-alkylen, Cyanalkylen, A-C(=NOH) oder A-C(=NNH₂),
- A: Alkyl mit 1-6 C-Atomen,
- -alkylen: Alkylen mit 1-6 C-Atomen und
- Ac: Alkanoyl mit 1-8 C-Atomen oder Aroyl mit 7-11 C-Atomen
bedeuten,
sowie deren Salze.

Ähnliche Verbindungen sind bekannt aus der EP-A1-76075 und der EP-A1-173848.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in Regel bei niedrigeren Dosen ein selektiver Angriff am Coronarsystem, bei höheren ein blutdrucksenkender Effekt beobachtet werden kann. Am Coronarsystem treten z. B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt (CH₂)ₙ-, wobei n 3,4, 5 oder 6 bedeutet.

Die Gruppe "alkylen" steht vorzugsweise für -CH₂- oder -CH₂CH₂-.

AC ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl.

R³ und R⁴ sind bevorzugt zusammen eine Bindung. Falls R⁴ H bedeutet, ist R³ bevorzugt OH, O-CHO oder O-COCH₃.

R⁵ ist bevorzugt unsubstituiertes 2-Hydroxy-4-pyridyl-oxy, 6-Hydroxy-3-pyridazinyl-oxy, 1,6-Dihydro-1-methyl- oder 1,6-Dihydro-1-ethyl-6-oxo-3-pyridazinyl-oxy, weiterhin 2-, 3- oder 4-Pyridyl-oxy.

In R⁶ und R⁷ bedeuten vorzugsweise:
- A:: Methyl, ferner Ethyl;
- AO:: Methoxy, ferner Ethoxy;
- ACO:: Acetyl, ferner Propionyl;
- ACS:: Thioacetyl, ferner Thiopropionyl;
- AOOC:: Methoxycarbonyl, ferner Ethoxycarbonyl;
- AO-CS:: Methoxy-thiocarbonyl, ferner Ethoxythiocarbonyl;
- ACOO:: Acetoxy, ferner Propionoxy;
- ACSO:: Thio(no)acetoxy, ferner Thio(no)propionoxy;
- Hydroxy-alkylen:: Hydroxymethyl oder 1- oder 2-Hydroxyethyl;
- Mercapto-alkylen:: Mercaptomethyl oder 1- oder 2-Mercaptoethyl;
- NHA:: Methylamino, ferner Ethylamino;
- NA₂:: Dimethylamino, ferner Diethylamino;
- ASO:: Methylsulfinyl, ferner Ethylsulfinyl;
- ASO₂:: Methylsulfonyl, ferner Ethylsulfonyl;
- AO-SO:: Methoxy-sulfinyl, ferner Ethoxy-sulfinyl;
- AO-SO₂:: Methoxy-sulfonyl, ferner Ethoxy-sulfonyl;
- Ac-NH:: Acetamido, ferner Formamido, Propionamido oder Benzamido;
- AO-CO-NH:: Methoxycarbonylamino, ferner Ethoxycarbonylamino;
- HANSO:: Methylaminosulfinyl, ferner Ethylaminosulfinyl
- A₂NSO:: Dimethylaminosulfinyl, ferner Diethylaminosulfinyl;
- HANSO₂:: Methylaminosulfonyl, ferner Ethylaminosulfonyl;
- A₂NSO₂:: Dimethylaminosulfonyl, ferner Diethylaminosulfonyl;
- HANCO:: N-Methylcarbamoyl, ferner N-Ethylcarbamoyl;
- A₂NOC:: N,N-Dimethylcarbamoyl, ferner N,N-Diethylcarbamoyl;
- HANCS:: N-Methyl-thiocarbamoyl, ferner N-Ethylthiocarbamoyl;
- A₂NCS:: N,N-Dimethyl-thiocarbamoyl, ferner N,N-Diethyl-thiocarbamoyl;
- ASONH:: Methylsulfinylamino, ferner Ethylsulfinylamino;
- ASO₂NH:: Methylsulfonylamino, ferner Ethylsulfonylamino;
- AOSONH:: Methoxysulfinylamino, ferner Ethoxysulfinylamino;
- AOSO₂NH:: Methoxysulfonylamino, ferner Ethoxysulfonylamino;
- ACO-alkylen:: 2-Oxopropyl, 2-Oxobutyl, 3-Oxobutyl, 3-Oxopentyl;
- Nitro-alkylen:: Nitromethyl, 1- oder 2-Nitroethyl;
- Cyan-alkylen:: Cyanmethyl, 1- oder 2-Cyanethyl;
- A-C(= NOH):: 1-Oximinoethyl, ferner 1-Oximinopropyl;
- A-C(= NNH₂):: 1-Hydrazonoethyl, ferner 1-Hydrazonopropyl.

Die Reste R⁶ und R⁷ stehen Vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten R⁶ und R⁷ ist Vorzugsweise der eine H, während der andere von H Verschieden ist. Dieser andere Rest steht vorzugsweise in 6-Stellung, aber auch in 5-, 7- oder 8-Stellung, und ist vorzugsweise CN oder NO₂, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, CF₃, H₂NCO, H₂NCS oder NH₂.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ig ausgedrückt werden, die der Formel I entsprechen und worin die nicht näher bezeichneten Reste die bei der Formel I angegebene Bedeutung haben, worin jedoch
in Ia
   - R¹ und R²: jeweils A bedeuten;
in Ib
   - R¹ und R²: jeweils CH₃ bedeuten;
in Ic
   - R¹ und R²: Zusammen Alkylen mit 3-6 C-Atomen bedeuten;
in Id
   - R⁵: 2-Hydroxy-4-pyridyl-oxy, 6-Hydroxy-3-pyridazinyl-oxy,1,6-Dihydro-1-methyl-6-oxo-3-pyridazinyl-oxy oder 1,6-Dihydro-1-ethyl-6-oxo-3-pyridazinyl-oxy bedeutet;
in Ie
   - R⁵: 6-Hydroxy-3-pyridazinyl-oxy bedeutet;
in If
   - R¹ und R² und R⁵: jeweils CH₃ und 2-Hydroxy-4-pyridyl-oxy, 6-Hydroxy-3-pyridazinyl-oxy, 1,6-Dihydro-1-methyl-6-oxo-3-pyridazinyloxy oder 1,6-Dihydro-1-ethyl-6-oxo-3-pyridazinyl-oxy bedeuten;
in Ig
   - R¹ und R² und R⁵: jeweils CH₃ und 6-Hydroxy-3-pyridazinyl-oxy bedeuten.

Weiterhin sind bevorzugt Verbindungen der Formeln I' sowie Ia' bis Ig', die den Formeln I sowie Ia bis Ig entsprechen, worin jedoch jeweils zusätzlich R³ OH, OCHO oder OCOCH₃ und R⁴ H bedeuten.

Weiterhin sind bevorzugt Verbindungen der Formeln I'' sowie Ia'' bis Ig'', die den Formeln I sowie Ia bis Ig entsprechen, worin jedoch jeweils zusätzlich R³ und R⁴ zusammen eine Bindung bedeuten.

Ferner sind bevorzugt Verbindungen der Formeln I, I', I'', Ia bis Ig, Ia' bis Ig' sowie Ia'' bis Ig'', worin jeweils zusätzlich
(a)
   - R⁶: von H verschieden ist und
   - R⁷: H bedeutet;
(b)
   - R⁶: von H verschieden ist und in 6-Stellung steht und
   - R⁷: H bedeutet;
(c)
   - R⁶: NO₂, CN, CHO, ACO, HOOC, AOOC, ACOO, F, Cl, Br, J, CF₃, H₂NCO, H₂NCS oder NH₂ und
   - R⁷: H bedeutet;
(d)
   - R⁶: NO₂, CN, CHO, ACO, HOOC, AOOC, ACOO, F, Cl, Br, J, CF₃, H₂NCO, H₂NCS oder NH₂ bedeutet und in 6-Stellung steht und
   - R⁷: H bedeutet;
(e)
   - R⁶: NO₂, CN, CHO, CH₃CO, CH₃OOC,C₂H₅OOC oder CH₃COO und
   - R⁷: H bedeutet;
(f)
   - R⁶: NO₂, CN, CHO, CH₃CO, CH₃OOC,C₂H₅OOC oder CH₃COO bedeutet und in 6-Stellung steht und
   - R⁷: H bedeutet;
(g)
   - R⁶: NO₂ oder CN und
   - R⁷: H bedeutet;
(h)
   - R⁶: NO₂ oder CN bedeutet und in 6-Stellung steht und
   - R⁷: H bedeutet;
(i)
   - R⁶: CN und
   - R⁷: H bedeutet;
(j)
   - R⁶: CN bedeutet und in 6-Stellung steht und
   - R⁷: H bedeutet.

Insbesondere sind bevorzugt Verbindungen der Formeln I, I', I'', Ia bis Ig, Ia' bis Ig', Ia'' bis Ig'' sowie der übrigen vorstehend als bevorzugt gekennzeichneten Gruppen von Verbindungen, worin zusätzlich R⁸ CH₃ bedeutet.

Im übrigen haben vor- und nachstehend die Reste R¹ bis R⁸, A, "alkylen" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichent, daß man ein 3,4-Epoxychroman der Formel II 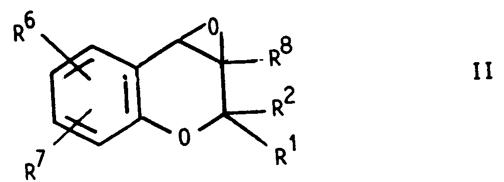
worin
R¹, R², R⁶, R⁷ und R⁸ die bei Formel I angegebene Bedeutung haben,
mit einer Verbindung der Formel III

R⁵-H III

worin R⁵ die bei Formel I angegebene Bedeutung hat oder mit einem ihrer reaktionsfähigen Derivate umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und /oder R⁷ in andere Reste R³, R⁵, R⁶ und/oder R⁷ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegeben Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Die Ausgangsstoffe der Formel III sind in der Regel bekannt. Sofern sie nicht bekannt sind, können sie nach an sich bekannten Methoden hergestellt werden. Die Ausgangsstoffe der Formel II sind erhältlich durch Umsetzung von 2-Hydroxyacetophenonen der Formel 2-HO-R⁶R⁷C₆H₂-COCH₃ mit Ketonen der Formel R¹-CO-R² zu entsprechenden 4-Chromanonen der Formel Va, 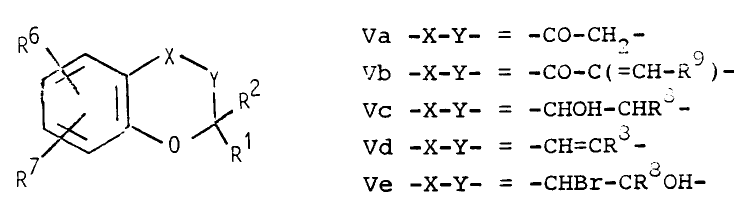
Kondensation mit Aldehyden der Formel R⁹-CHO (R⁹ = Alkyl mit 1-5 C-Atomen) zu 3-Alkyliden-4-chromanonen der Formel Vb, Reduktion z.B. mit NaBH₄ zu 3-Alkyl-4-chromanolen der Formel Vc, Dehydratisierung z.B. mit p-Toluolsulfonsäure, zu Chromenen der Formel Vd und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung, zunächst die Bromhydrine der Formel Ve herstellen und aus diesen anschließend mit einer Base, z.B. Natronlauge, HBr abspalten.

Man kann die Chromene der Formel Vd auch erhalten durch Kondensation von Salicylaldehyden der Formel 2-HO-R⁶R⁷-C₆H₂-CHO mit Ketonen der Formel R¹-CO-CH₂-R⁸ zu Hydroxyketonen der Formel 2-HO-R⁶R⁷C₆H₂-CH=CR⁸-CO-R¹, Umsetzung mit Organo-Li-Verbindungen der Formel R²-Li und nachfolgende Hydrolyse zu Diolen der Formel 2-HO-R⁶R⁷C₆H₂-CH=CR⁸-CR¹R²-OH und Cyclisierung unter Wasserabspaltung.

Als reaktionsfähige Derivate von III eignen sich die entsprechenden Salze, z. B. die Na- oder K-Salze, die auch in situ entstehen können.

Bei der Umsetzung von II mit III können verschiedene Produkte der Formel I entstehen, abhängig insbesondere von der Struktur der Ausgangsstoffe III und von den Reaktionsbedingungen.

Beispielsweise ist die Bildung von Verbindungen der Formel I begünstigt, wenn die Verbindung III zusätzlich zu der Lactam- bzw. Lactimgruppierung mindestens eine OH-Gruppe als Substituenten enthält und/oder wenn man unter relativ milden Bedingungen, z.B. in Gegenwart einer schwachen Base wie Pyridin in einem Alkohol, arbeitet. Beispielsweise entstehen aus 2,2,3-Trimethyl-3,4-epoxy-6-cyan-chroman ("IIa") und 1H-2-Pyridon mit NaH im DMSO überwiegend 2,2,3-Trimethyl-4-(1H-2-pyridon-1-yl)-6-cyan-2H-chromen ("A") und 2,2,3-Trimethyl-4-(1H-2-pyridon-1-yl)-6-cyan-chroman-3-ol ("B"), mit Pyridin in Ethanol dagegen "B" und 2,2,3-Trimethyl-4-(2-pyridyl-oxy)-6-cyan-chroman-3-ol zu etwa gleichen Teilen. Aus 2,4-Dihydroxypyridin (= 4-Hydroxy-1H-2-pyridon) und IIa entstehen in Pyridin/Ethanol 2,2,3-Trimethyl-4-(2-hydroxy-4-pyridyl-oxy)-6-cyan-chroman-3-ol und 2,2,3-Trimethyl-4-(4-hydroxy-1H-2-pyridon-1-yl)-6-cyan-chroman-3-ol im Mengenverhältnis von etwa 9:1.

Eine Optimierung der Reaktionsbedingungen gelingt leicht in jedem Einzelfall. Die Reaktionsprodukte können ohne Schwierigkeiten getrennt und isoliert werden, z.B. durch Kristallisation und/oder Chromatographie.

Es ist zweckmäßig, in Gegenwart einer Base zu arbeiten. Als Basen eignen sich z. B. Alkalimetall- oder Erdalkalimetall-hydroxide, -hydride oder auch -amide wie NaOH, KOH, Ca(OH)₂, NaH, KH, CaH₂, NaNH₂, KNH₂, ferner organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II kann auch in situ hergestellt werden, z. B. durch Einwirkung einer Base auf das entsprechende Bromhydrin Ve.

Eine Verbindung der Formel I, worin R³ = OH und R⁴ = H ist, kann durch Behandeln mit einem Dehydratisierungsmittel in eine Verbindung der Formel I, worin R³ und R⁴ zusammen eine Bindung bedeuten, umgewandelt werden. Das gelingt z. B. durch Einwirkung einer der angegebenen Basen, z. B. NaOH, KOH oder NaH, in einem der angegebenen Lösungsmittel, z. B. Tetrahydrofuran, Dioxan oder DMSO, bei Temperaturen zwischen 0 und 150 °.

Weiterhin kann man in einer Verbindung der Formel I einen oder mehrere der Reste R³, R⁵, R⁶ und/oder R⁷ in andere Reste R³, R⁵, R⁶ und/oder R⁷ umwandeln.

Beispielsweise ist es möglich, daß man ein H-Atom mittels einer Halogenierung durch ein Halogenatom oder einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z. B. mit Raney-Nickel in Wasser/Essigsäure/Pyridin in Gegenwart von Natriumphosphat) in eine Formylgruppe oder (z. B. mit KOH in tert.-Butanol) in eine Carbamoylgruppe oder (z. B. mit H₂S kin Pyridin/Triethylamin) in eine Thiocarbamoylgruppe umwandelt und/oder einen substituierten oder unsubstituierten 1H-2-Pyridon-1-ylrest (z. B. mit P₂S₅ oder mit Lawesson-Reagenz in Toluol) in den entsprechenden 1H-2-Thiopyridon-1-ylrest umwandelt.

Eine Nitrierung gelingt unter üblichen Bedingungen, z. B. mit einem Gemisch aus konzentrierter HNO₃ und konzentrierter H₂SO₄ bei Temperaturen zwischen 0 und 30 °. Falls mindestens einer der Substituenten R⁶ und R⁷ eine elektronegative Gruppe wie CN oder NO₂ bedeutet, erfolgt die Nitrierung überwiegend am Rest R⁵; andernfalls erhält man in der Regel Gemische, bei denen die Nitrogruppen am Rest R⁵ oder am Chromanring stehen können.

Analoges gilt für die Halogenierung, die z. B. mit elementarem Chlor oder Brom in einem der üblichen inerten Lösungsmittel bei Temperaturen zwischen etwa 0 und 30 ° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z. B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z. B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungsmittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z. B. Chloride oder Bromide) oder Anhydride von Carbonsäuren der Formel Ac-OH, z. B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich.Man acyliert zweckmäßig in Gegenwart oder Abwesenheit eines inerten Lösungsmittels, z. B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern.So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäure, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z. B. Verbindungen der Formel I, worin R¹ = R², R³ = OH und R⁴ = H ist, zwei chirale Zentren; bei der Herstellung durch Reaktion von II mit III entsteht jedoch ganz überwiegend nur ein Racemat mit trans-Stellung der Substituenten R³ = OH und R⁵. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z. B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, R³ = OH) können ferner mit Hilfe chiraler Acylierungsreagenzien, z. B. D- oder L-α-Methylbenzylisocyanat, verestert und dann getrennt werden (vgl. EP-A1-120428). Die verschiedenen Formen der Diastereomeren können in an sich bekannter Weise, z. B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, z B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern verabreicht.

Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Erfindung betrifft auch das neue Zwischenprodukt 2,2,3-Trimethyl-3,4-epoxy-6-cyan-chroman.

In den nachfolgenden Beispielen bedeutet "übliche Aufarbeitung":
Man gibt, falls erforderlich, Wasser hinzu, extrahiert mit einem organischen Lösungsmitte wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel (a)

### Herstellung des Zwischenprodukts 2,2,3-Trimethyl-3,4-epoxy-6-cyan-chroman ("IIa"):

(a) Ein Gemisch von 81 g 3-Acetyl-4-hydroxybenzonitril, 48 ml Aceton, 11,8 ml Pyrrolidin und 300 ml Toluol wird 1 Std. bei 20° stehengelassen, dann 2 Std. am Wasserabscheider gekocht und abgekühlt. Nach üblicher Aufarbeitung erhält man 2,2-Dimethyl-6-cyan-4-chromanon, F. 119-120°.
(b) Eine Lösung von 24 g des Chromanons, 12 g Paraformaldehyd und 24 ml Piperidin in 300 ml Ethanol wird 3 Std. auf 70° erhitzt und eingedampft. Man nimmt den Rückstand in Dichlormethan/Petrolether 1:1 auf, filtriert über Kieselgel, dampft ein und erhält 2,2-Dimethyl-3-methylen-6-cyan-4-chromanon als instabiles Öl.
(c) Eine Lösung von 25 g des vorstehenden Chromanons in 500 ml Methanol wird mit 6 g NaBH₄ versetzt, 1 Std. bei 20° gerührt und eingedampft. Nach üblicher Aufarbeitung erhält man 2,2,3-Trimethyl-6-cyan-4-chromanol, Isomerengemisch, ölig.
(d) Eine Lösung von 27 g des vorstehenden Gemischs und 1,2 g p-Toluolsulfonsäure in 400 ml Toluol wird 3 Std. am Wasserabscheider gekocht. Man dampft ein, löst in Dichlormethan/Petrolether 1:1, filtriert über Kieselgel, dampft erneut ein und erhält 2,2,3-Trimethyl-6-cyan-2H-chromen, F. 55°.
(e) Eine Lösung von 6,4 g m-Chlorperbenzoesäure in 40 ml Dichlormethan wird unter Rühren zu einer Lösung von 6,8 g des vorstehenden Chromens in 100 ml Dichlormethan zugetropft. Man rührt noch 16 Std., filtriert, gibt verdünnte Natronlauge zu, arbeitet wie üblich auf und erhält 2,2,3-Trimethyl-3,4-epoxy-6-cyan-chroman (IIa), F. 118°. Die Enantiomeren von IIa sind erhältlich durch Reaktion von 2,2,3-Trimethyl-6-cyan-2H-chromen mit N-Bromsuccinimid zu 2,2,3-Trimethyl-3-brom-6-cyan-chroman-4-ol, Veresterung mit (+)- oder (-)-Camphandsäurechlorid zu den diastereomeren Camphansäureestern, Trennung der Enantiomeren durch Kristallisation oder Chromatographie und Behaldeln mit Base, wobei Verseifung und Ringschluß zum enantiomeren Epoxid IIa erfolgt.

### Beispiel 1

Ein Gemisch von 21,5 g IIa, 11,2 g 3,6-Pyridazindiol (= 3-Hydroxy-1H-6-pyridazinon), 12 ml Pyridin und 600 ml Ethanol wird 72 Std. gekocht. Man destilliert etwa 300 ml ab, kühlt ab, filtriert nicht umgesetztes 3,6-Pyridazindiol ab und dampft ein. Das erhaltene 2,2,3-Trimethyl-4-(6-hydroxy-3-pyridazinyl-oxy)-6-cyan-chroman-3-ol [= 2,2,3-Trimethyl-4-(1,6-dihydro-6-oxo-3-pyridazinyl-oxy)-6-cyan-chroman-3-ol] wird aus Isopropanol umkristallisiert. F. 240°.

Analog erhält man aus den entsprechenden 3,4-Epoxy-chromanen:
2,3-Dimethyl-4-(6-hydroxy-3-pyridazinyl-oxy)-chroman-3-ol
2,3-Dimethyl-4-(6-hydroxy-3-pyridazinyl-oxy)-6-cyan-chroman-3-ol
2,3-Dimethyl-4-(6-hydroxy-3-pyridazinyl-oxy)-6-nitro-chroman-3-ol
2,3-Dimethyl-2-ethyl-4-(6-hydroxy-3-pyridazinyl-oxy)-6-cyan-chroman-3-ol
2,2-Diethyl-3-methyl-4-(6-hydroxy-3-pyridazinyl-oxy)-6-cyan-chroman-3-ol
2,2-Trimethylen-3-methyl-4-(6-hydroxy-3-pyridazinyl-oxy)-6-cyan-chroman-3-ol
2,2,3-Trimethyl-4-(6-hydroxy-3-pyridazinyl-oxy)-chroman-3-ol
2,2,3-Trimethyl-4-(6-hydroxy-3-pyridazinyl-oxy)-6-methyl-chroman-3-ol
2,2,3-Trimethyl-4-(6-hydroxy-3-pyridazinyl-oxy)-6-methoxy-chroman-3-ol
2,2,3-Trimethyl-4-(6-hydroxy-3-pyridazinyl-oxy)-6-thioacetyl-chroman-3-ol
2,2,3-Trimethyl-4-(6-hydroxy-3-pyridazinyl-oxy)-6-methoxy-thiocarbonyl-chroman-3-ol
2,2,3-Trimethyl-4-(6-hydroxy-3-pyridazinyl-oxy)-6-thio(no)-acetoxy-chroman-3-ol
2,2,3-Trimethyl-4-(6-hydroxy-3-pyridazinyl-oxy)-6-hydroxymethyl-chroman-3-ol
2,2,3-Trimethyl-4-(6-hydroxy-3-pyridazinyl-oxy)-6-dimethylamino-chroman-3-ol
2,2,3-Trimethyl-4-(6-hydroxy-3-pyridazinyl-oxy)-6-brom-chroman-3-ol
2,2,3-Trimethyl-4-(6-hydroxy-3-pyridazinyl-oxy)-6-jod-chroman-3-ol
2,2,3-Trimethyl-4-(6-hydroxy-3-pyridazinyl-oxy)-6-methylsulfinyl-chroman-3-ol
2,2,3-Trimethyl-4-(6-hydroxy-3-pyridazinyl-oxy)-6-methylsulfonyl-chroman-3-ol
2,2-Dimethyl-3-hexyl-4-(6-hydroxy-3-pyridazinyl-oxy)-6-cyan-chroman-3-ol.

### Beispiel 2

Ein Gemisch von 6,5 g IIa, 3,1 g Pyridon, 3 ml Pyridin un 100 ml Ethanol wird 72 Std. gekocht. Nach Abkühlen und üblicher Aufarbeitung chromatographiert man an Kieselgel. Mit Dichlormethan/Petrolether (85:15) wird 2,2,3-Trimethyl-4-(2-pyridyl-oxy)-6-cyan-chroman-3-ol (F. 105-107°) eluiert, anschließend mit Dichlormethan/Ethylacetat (85:15) 2,2,3-Trimethyl-4-(1H-2-pyridon-1-yl)-3-cyan-chroman-3-ol ("B"), F. 185-186°; Mengenverhältnis etwa 1:1. Die Verbiundung "B" wird nicht beansprucht.

### Beispiel 3

Ein Gemisch von 21,5 g IIa, 11,1 g 2,4-Dihydroxypyridin (= 4-Hydroxy-1H-2-pyridon), 12 ml Pyridin und 360 ml Ethanol wird 48 Stunden gekocht. Nach Abkühlen und üblicher Aufarbeitung chromatographiert man an Kieselgel. Mit Dichlormethan/Ethylacetat (85:15) wird 2,2,3-Trimethyl-4-(4-hydroxy-1H-2-pyridon-1-yl)-6-cyan-chroman-3-ol (F. 225-227°) eluiert, anschließend mit Ethylacetat/Methanol (90:10) 2,2,3-Trimethyl-4-(1,2-dihydro-2-oxo-4-pyridyloxy)-6-cyan-chroman-3-ol [2,2,3-Trimethyl-4-(2-hydroxy-4-pyridyl-oxy)-6-cyan-chroman-3-ol, F. 198-200°], Mengenverhältnis etwa 1:9.
Das erstgenannte Reaktionsprodukt wird nicht beansprucht.

### Beispiel 4

Analog Beispiel 1 erhält man aus IIa bzw. den entsprechenden 2,2,3-Trimethyl-3,4-epoxy-chromanen
mit 3-Hydroxypyridin:
2,2,3-Trimethyl-4-(3-pyridyl-oxy)-6-cyan-chroman-3-ol;
mit 4-Hydroxypyridin:
2,2,3-Trimethyl-4-(4-pyridyl-oxy)-6-cyan-chroman-3-ol;
mit 3-Hydroxypyridazin:
2,2,3-Trimethyl-4-(3-pyridazinyl-oxy)-6-cyan-chroman-3-ol;
mit 4-Hydroxypyrimidin:
2,2,3-Trimethyl-4-(3-pyrimidinyl-oxy)-6-cyan-chroman-3-ol;
mit 2-Hydroxypyrazin:
2,2,3-Trimethyl-4-(2-pyrazinyl-oxy)-6-cyan-chroman-3-ol;
mit 2,4-Dihydroxypyridin:
2,2,3-Trimethyl-4-(2-hydroxy-4-pyridyl-oxy)-6-nitro-chroman-3-ol
2,2,3-Trimethyl-4-(2-hydroxy-4-pyridyl-oxy)-6-brom-chroman-3-ol
2,2,3-Trimethyl-4-(2-hydroxy-4-pyridyl-oxy)-6-methoxycarbonyl-chroman-3-ol;
mit 2,3-Dihydroxypyridin:
2,2,3-Trimethyl-4-(2-hydroxy-3-pyridyl-oxy)-6-cyan-chroman-3-ol;
mit 2,5-Dihydroxypyridin:
2,2,3-Trimethyl-4-(2-hydroxy-5-pyridyl-oxy)-6-cyan-chroman-3-ol;
mit 4,6-Dihydroxypyrimidin:
2,2,3-Trimethyl-4-(6-hydroxy-4-pyrimidinyl-oxy)-6-cyan-chroman-3-ol;
mit 3,6-Dihydroxypyridazin:
2,2,3-Trimethyl-4-(6-hydroxy-3-pyridazinyl-oxy)-6-nitro-chroman-3-ol, F. 223-225
2,2-Tetramethylen-3-methyl-4-(6-hydroxy-3-pyridazinyl-oxy)-6-cyan-chroman-3-ol
2,2-Pentamethylen-3-methyl-4-(6-hydroxy-3-pyridazinyl-oxy)-6-cyan-chroman-3-ol, kein F. bis 275°
2,2,3-Trimethyl-4-(6-hydroxy-3-pyridazinyl-oxy)-6-brom-chroman-3-ol
2,2,3-Trimethyl-4-(6-hydroxy-3-pyridazinyl-oxy)-6-methoxycarbonyl-chroman-3-ol.

### Beispiel 5

Ein Gemisch von 327 mg 2,2,3-Trimethyl-4-(1,6-dihydro-6-oxo-3-pyridazinyl-oxy)-6-cyan-chroman-3-ol, 20 ml Aceton, 400 mg K₂CO₃ und 0,2 ml Dimethylsulfat wird 2 Stunden gekocht. Man filtriert, dampft ein und chromatographiert an Kieselgel. Mit Ethylacetat/Methanol (9:1) erhält man 2,2,3-Trimethyl-4-(1,6-dihydro-1-methyl-6-oxo-3-pyridazinyl-oxy)-6-cyan-chroman-3-ol, F.197-199°.

Analog erhält man durch Alkylierung:
2,2,3-Trimethyl-4-(1,2-dihydro-1-methyl-2-oxo-4-pyridyl-oxy)-6-cyan-chroman-3-ol
2,2,3-Trimethyl-4-(1,2-dihydro-1-ethyl-2-oxo-4-pyridyl-oxy)-6-cyan-chroman-3-ol
2,2,3-Trimethyl-4-(1,6-dihydro-1-ethyl-6-oxo-3-pyridazinyl-oxy)-6-cyan-chroman-3-ol, F. 166-168°.

Die nachstehenden Beispiele betreffen pharmazeutische Zubereitungen, die Verbindungen der Formel I oder ihre physiologisch unbedenklichen Salze enthalten.

### Beispiel A Kapseln

Man füllt 1 kg 2,2,3-Trimethyl-4-(6-hydroxy-3-pyridazinyl-oxy)-6-cyan-chroman-3-ol in üblicher Weise in Hartgelatinekapseln, so daß jede Kapsel 0,5 mg Wirkstoff enthält.

### Beispiel B Ampullen

Eine Lösung von 1 kg 2,2,3-Trimethyl-4-(2-hydroxy-4-pyridyl-oxy)-6-cyan-chroman-3-ol in einem Gemisch von 20 l 1,2-Propandiol und 10 l zweifach destilliertem Wasser wird steril filtriert, in Ampullen abgefüllt und steril verschlossen. Jede Ampulle enthält 0,1 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Patentansprüche (Patentansprüche für folgende(n) Vertragsstaat(en): AT, BE, CH, DE, FR, GB, IT, LI, LU, NL, SE)

1. Chromanderivate der Formel I 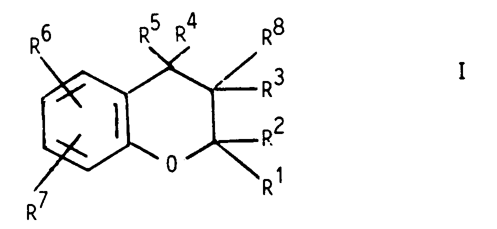 worin
R¹ und R⁸ jeweils A,
R² H oder A,
R¹ und R² zusammen auch Alkylen mit 3-6 C-Atomen,
R³ OH oder OAc,
R⁴ H,
R³ und R⁴ zusammen auch eine Bindung,
R⁵ einen unsubstituierten oder ein- oder zweifach durch A, F, Cl, Br, J, OH, OA, OAc, NO₂, NH₂, AcNH, HOOC und/oder AOOC substituierten Pyridyl-oxy-, Pyridazinyl-oxy-, Pyrimidinyl-oxy-, Pyrazinyl-oxy-, Oxo-dihydro-pyridyl-oxy-, Oxo-dihydro-pyridazinyl-oxy-, Oxo-dihydro-pyrimidinyl-oxy- oder Oxo-dihydro-pyrazinyl-oxy-rest,
R⁶ und R⁷ jeweils H, A, HO, AO, CHO, ACO,ACS, HOOC, AOOC, AO-CS,ACOO, A-CS-O, Hydroxy-alkylen, Mercapto-alkylen, NO₂, NH₂, NHA, NA₂, CN,F,Cl,Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylen, Nitro-alkylen, Cyan-alkylen, A-C(=NOH) oder A-C(=NNH₂),
A Alkyl mit 1-6 C-Atomen,
-alkylen Alkylen mit 1-6 C-Atomen und
Ac Alkanoyl mit 1-8 C-Atomen oder Aroyl mit 7-11 C-Atomen
bedeuten,
sowie deren Salze.

2. a) 2,2,3-Trimethyl-4-(2-hydroxy-4-pyridyl-oxy)-6-cyan-chroman-3-ol;
b) 2,2,3-Trimethyl-4-(6-hydroxy-3-pyridazinyl-oxy)-6-cyan-chroman-3-ol;
c) 2,2,3-Trimethyl-4-(1,6-dihydro-1-methyl-6-oxo-3-pyridazinyl-oxy)-6-cyan-chroman-3-ol;
d) 2,2,3-Trimethyl-4-(1,6-dihydro-1-ethyl-6-oxo-3-pyridazinyl-oxy)-6-cyan-chroman-3-ol.

3. Verfahren zur Herstellung von Chromanderivaten der Formel I nach Patentanspruch 1, dadurch gekennzeichnet, daß man ein 3,4-Epoxychroman der Formel II 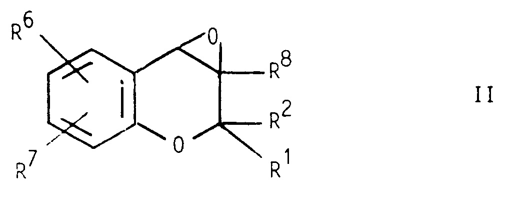 worin
R¹, R², R⁶ , R⁷ und R⁸ die bei Formel I angegebene Bedeutung haben,
mit einer Verbindung der Formel III
R⁵-H III
worin R⁵ die bei Formel I angegebene Bedeutung hat oder mit einem ihrer reaktionsfähigen Derivate umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und /oder R⁷ in andere Reste R³, R⁵, R⁶ und/oder R⁷ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I nach Patentanspruch 1 und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I nach Patentanspruch 1 und/oder einem ihrer physiologisch unbedenklichen Salze.

6. Verbindungen der Formel I nach Patentanspruch 1 zur Bekämpfung von Krankheiten.

7. Verwendung von Verbindungen der Formel I nach Patentanspruch 1 zur Herstellung eines Arzneimittels.

8. 2,2,3-Trimethyl-3,4-epoxy-6-cyan-chroman.

## Patentansprüche (Patentansprüche für folgende(n) Vertragsstaat(en): ES)

1. Verfahren zur Herstellung von Chromanderivaten der Formel I 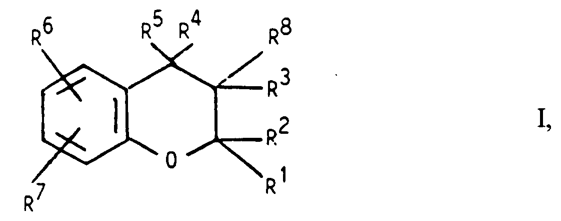 worin
R¹ und R⁸ jeweils A,
R² H oder A,
R¹ und R² zusammen auch Alkylen mit 3-6 C-Atomen,
R³ OH oder OAc,
R⁴ H
R³ und R⁴ zusammen auch eine Bindung,
R⁵ einen unsubstituierten oder ein- oder zweifach durch A, F, Cl, Br, I, OH, OA, OAc, NO₂, NH₂, AcNH, HOOC und/oder AOOC substituierten Pyridyl-oxy-, Pyridazinyl-oxy-, Pyrimidinyl-oxy-, Pyrazinyl-oxy-, Oxo-dihydropyridyl-oxy-, Oxo-dihydropyridazinyl-oxy-, Oxo-dihydropyrimidinyl-oxy- oder Oxo-dihydropyrazinyl-rest,
R⁶ und R⁷ jeweils H, A, HO, AO, CHO, ACS, HOOC, AOOC, AO-CS, ACOO, A-CS-O, Hydroxyalkylen, Mercaptoalkylen, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS,HANCS, A₂NCS, ASONH, ASO₂NH, AOSONH, AOSO₂NH, ACO-alkylen, Nitroalkylen, Cyanalkylen, A-C (=NOH) oder A-C (=NNH₂),
A Alkyl mit 1-6 C-Atomen,
-alkylen Alkylen mit 1-6 C-Atomen,
Ac Alkanoyl mit 1-8 Atomen oder Aroyl mit 7-11 C-Atomen,
bedeuten,
sowie deren Salze, dadurch gekennzeichnet, daß man ein 3,4-Epoxychroman der Formel II 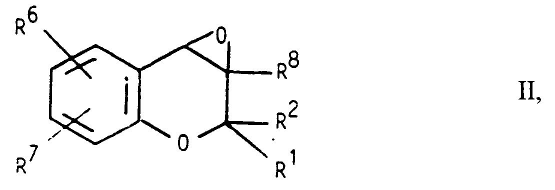 worin R¹, R², R⁶, R⁷ und R⁸ die bei Formel I angegebene Bedeutung haben, mit einer Verbindung der Formel III
R⁵-H III,
worin R⁵ die bei Formel I angegebene Bedeutung hat oder mit einem ihrer reaktionsfähigen Derivate umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und/oder R⁷ in andere Reste R³, R⁵, R⁶ und/oder R⁷ umwandelt und/oder man eine basische Verbindung der Formel I durch Behandeln mit einer Säure in eines ihrer Säureadditionssalze umwandelt.

2. Verfahren zur Herstellung pharmazeutischer Zubereitungen, dadurch gekennzeichnet, daß man eine Verbindung der Formel I nach Patentanspruch 1 und/oder eines ihrer physiologischen unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

## Claims (Claims for the following Contracting State(s): AT, BE, CH, DE, FR, GB, IT, LI, LU, NL, SE)

1. Chroman derivatives of the formula I 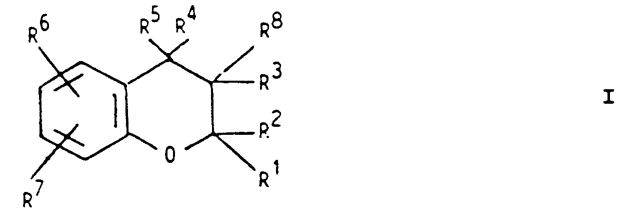 in which
R¹ and R⁸ are in each case A,
R² is H or A,
R¹ and R² together are also alkylene having 3-6 C atoms,
R³ is OH or OAc,
R⁴ is H,
R³ and R⁴ together are also a bond,
R⁵ is a pyridyloxy, pyridazinyloxy, pyrimidinyloxy, pyrazinyloxy, oxodihydropyridyloxy, oxodihydropyridazinyloxy, oxodihydropyrimidinyloxy or oxodihydropyrazinyloxy radical which is unsubstituted or monosubstituted or disubstituted by A, F, Cl, Br, I, OH, OA, OAc, NO₂, NH₂, AcNH, HOOC and/or AOOC,
R⁶ and R⁷ are in each case H, A, HO, AO, CHO, ACO, ACS, HOOC, AOOC, AO-CS, ACOO, A-CS-O, hydroxy-alkylene, mercapto-alkylene, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylene, nitro-alkylene, cyano-alkylene, A-C(=NOH) or A-C(=NNH₂),
A is alkyl having 1-6 C atoms,
-alkylene is alkylene having 1-6 C atoms and
Ac is alkanoyl having 1-8 C atoms or aroyl having 7-11 C atoms,
and their salts.

2. a) 2,2,3-trimethyl-4-(2-hydroxy-4-pyridyloxy)-6-cyanochroman-3-ol;
b) 2,2,3-trimethyl-4-(6-hydroxy-3-pyridazinyloxy)-6-cyanochroman-3-ol;
c) 2,2,3-trimethyl-4-(1,6-dihydro-1-methyl-6-oxo-3-pyridazinyloxy)-6-cyanochroman-3-ol;
d) 2,2,3-trimethyl-4-(1,6-dihydro-1-ethyl-6-oxo-3-pyridazinyloxy)-6-cyanochroman-3-ol.

3. Process for the preparation of chroman derivatives of the formula I according to Patent Claim 1, characterized in that a 3,4-epoxychroman of the formula II 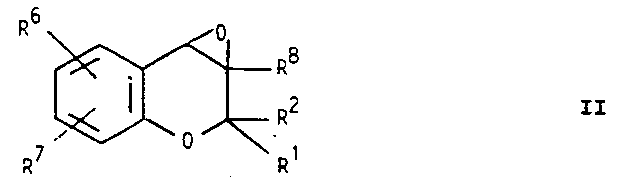 in which
R¹, R², R⁶, R⁷ and R⁸ have the meanings given in formula I, is reacted with a compound of the formula III
R⁵-H III
in which R⁵ has the meaning given in formula I
or with one of its reactive derivatives
and/or in that a compound of the formula I, in which R³ is OH and R⁴ is H, is dehydrated and/or in that one or more of the radicals R³, R⁵, R⁶ and/or R⁷ are converted into other radicals R³, R⁵, R⁶ and/or R⁷ in a compound of the formula I and/or in that a basic compound of the formula I is converted into one of its acid addition salts by treating with an acid.

4. Process for the production of pharmaceutical Preparations, characterized in that a compound of the formula I according to Patent Claim 1 and/or one of its Physiologically acceptable salts is brought into a suitable form for administration together with at least one solid, liquid or semi-liquid excipient or auxiliary and, if desired, in combination with one or more further active compound(s).

5. Pharmaceutical preparation, characterized in that it contains at least one compound of the formula I according to Patent Claim 1 and/or one of its physiologically acceptable salts.

6. Compounds of the formula I according to Patent Claim 1 for the control of diseases.

7. Use of compounds of the formula I according to Patent Claim 1 for the production of a medicament.

8. 2,2,3,-Trimethyl-3,4-epoxy-6-cyano-chroman.

## Claims (Claims for the following Contracting State(s): ES)

1. Process for the preparation of chroman derivatives of the formula I 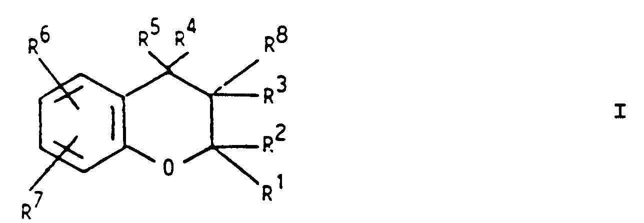 in which
R¹ and R⁸ are in each case A,
R² is H or A,
R¹ and R² together are also alkylene having 3-6 C atoms,
R³ is OH or OAc,
R⁴ is H,
R³ and R⁴ together are also a bond,
R⁵ is a pyridyloxy, pyridazinyloxy, pyrimidinyloxy, pyrazinyloxy, oxodihydropyridyloxy, oxodihydropyridazinyloxy, oxodihydropyrimidinyloxy or oxodihydropyrazinyl radical which is unsubstituted or monosubstituted or disubstituted by A, F, Cl, Br, I, OH, OA, OAc, NO₂, NH₂, AcNH, HOOC and/or AOOC,
R⁶ and R⁷ are in each case H, A, HO, AO, CHO, ACS, HOOC, AOOC, AO-CS, ACOO, A-CS-O, hydroxy-alkylene, mercapto-alkylene, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylene, nitro-alkylene, cyano-alkylene, A-C(=NOH) or A-C(=NNH₂),
A is alkyl having 1-6 C atoms,
-alkylene is alkylene having 1-6 C atoms,
Ac is alkanoyl having 1-8 atoms or aroyl having 7-11 C atoms,
and their salts, characterized in that a 3,4-epoxychroman of the formula II 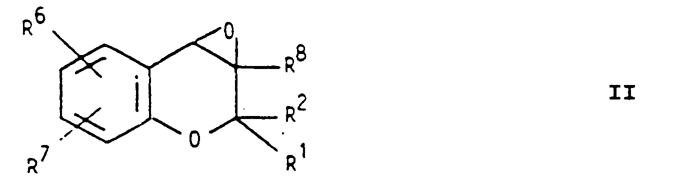 in which
R¹, R², R⁶, R⁷ and R⁸ have the meanings given in formula I, is reacted with a compound of the formula III
R⁵-H III
in which R⁵ has the meaning given in formula I
or with one of its reactive derivatives
and/or in that a compound of the formula I, in which R³ is OH and R⁴ is H, is dehydrated and/or in that one or more of the radicals R³, R⁵, R⁶ and/or R⁷ are converted into other radicals R³, R⁵, R⁶ and/or R⁷ in a compound of the formula I and/or a basic compound of the formula I is converted into one of its acid addition salts by treating with an acid.

2. Process for the production of pharmaceutical preparations, characterized in that a compound of the formula I according to Patent Claim 1 and/or one of its physiologically acceptable salts is brought into a suitable form for administration together with at least one solid, liquid or semi-liquid excipient or auxiliary and, if desired, in combination with one or more further active compound(s).

## Revendications (Revendications pour l'(les) Etat(s) contractant(s) suivant(s): AT, BE, CH, DE, FR, GB, IT, LI, LU, NL, SE)

1. Dérivés du chromane de formule I 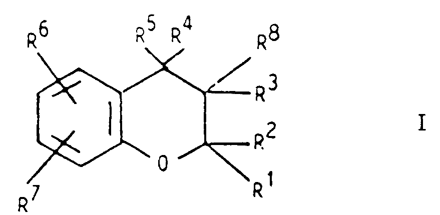 où
R¹ et R⁸ représentent A,
R² H ou A,
R¹ et R² représentent ensemble également un alkylène contenant 3 à 6 atomes de C,
R³ OH ou OAc,
R⁴ H,
R³ et R⁴ représentent ensemble également une liaison,
R⁵ un reste pyridyl-oxy, pyridazinyl-oxy, pyrimidinyl-oxy, pyrazinyl-oxy, oxo-dihydropyridyl-oxy, oxo-dihydropyridazinyl-oxy, oxo-dihydropyromidinyl-oxy ou oxo-dihydropyrazinyl-oxy non substitué ou mono- ou disubstitué par A, F, Cl, Br, I, OH, OA, OAc, NO₂, NH₂, AcNH, HOOC et/ou AOOC,
R⁶ et R⁷ représentent H, A, HO, AO, CHO, ACO, ACS, HOOC, AOOC, AO-CS, ACOO, A-CS-O, un hydroxyalkylène. un mercaptoalkylène, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylène, nitroalkylène, cyanoalkylène, A-C(=NOH) ou A-C(=NNH₂),
A un alkyle contenant 1 à 6 atomes de C,
-alkylène un alkylène contenant 1 à 6 atomes de C et
Ac un alcanoyle contenant 1 à 8 atomes de C ou un aroyle contenant 7 à 11 atomes de C,
ainsi que leurs sels.

2. a) le 2,2,3-triméthyl-4-(2-hydroxy-4-pyridyl-oxy)-6-cyano-chroman-3-ol ;
b) le 2,2,3-triméthyl-4-(6-hydroxy-3-pyridazinyl-oxy)-6-cyano-chroman-3-ol ;
c) le 2,2,3-triméthyl-4-(1,6-dihydro-1-méthyl-6-oxo-3-pyridazinyl-oxy)-6-cyano-chroman-3-ol ;
d) le 2,2,3-triméthyl-4-(1,6-dihydro-1-éthyl-6-oxo-3-pyridazinyl-oxy)-6-cyano-chroman-3-ol.

3. Procédé pour la préparation des dérivés du chromane de formule I selon la revendication 1, caractérisé en ce que l'on fait réagir un 3,4-époxychromane de formule II 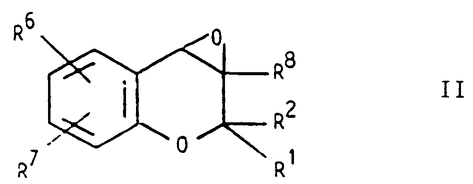 où
R¹, R², R⁶, R⁷ et R⁸ ont la signification indiquée pour la formule I,
avec un composé de formule III
R⁵-H III
où R⁵ a la signification indiquée pour la formule I ou avec l'un de ses dérivés réactifs,
et/ou en ce que l'on déshydrate un composé de formule I où R représente OH et R⁴ H et/ou en ce que l'on transforme dans un composé de formule I un ou plusieurs des restes R³, R⁵, R⁶ et/ou R⁷ en d'autres restes R³, R⁵, R⁶ et/ou R⁷ et/ou en ce que l'on transforme un composé basique de formule I par traitement avec un acide en l'un de ses sels d'addition d'acide.

4. Procédé pour la préparation de compositions pharmaceutiques, caractérisé en ce que l'on met sous une forme de dosage appropriée un composé de formule I selon la revendication 1 et/ou l'un de ses sels physiologiquement acceptables, associé à au moins un support ou un adjuvant solide, liquide ou semi-liquide et éventuellement en combinaison avec une ou plusieurs autre(s) substance(s) active(s).

5. Composition pharmaceutique, caractérisée en ce qu'elle contient au moins un composé de formule I selon la revendication 1 et/ou l'un de ses sels physiologiquement acceptables.

6. Composés de formule I selon la revendication 1 pour lutter contre les maladies.

7. Utilisation des composés de formule I selon la revendication 1 pour la préparation d'un médicament.

8. Le 2,2,3-triméthyl-3,4-époxy-6-cyano-chromane.

## Revendications (Revendications pour l'(les) Etat(s) contractant(s) suivant(s): ES)

1. Procédé pour la préparation des dérivés du chromane de formule I 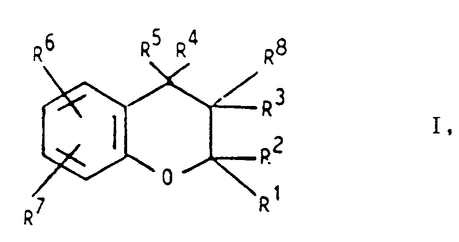 où
R¹ et R⁸ représentent A,
R² H ou A,
R¹ et R² représentent ensemble également un alkylène contenant 3 à 6 atomes de C,
R³ OH ou OAc,
R⁴ H,
R³ et R⁴ représentent ensemble également une liaison,
R⁵ un reste pyridyl-oxy, pyridazinyl-oxy, pyrimidinyl-oxy, pyrazinyl-oxy, oxo-dihydropyridyl-oxy, oxo-dihydropyridazinyl-oxy, oxo-dihydropyrimidinyl-oxy ou oxo-dihydropyrazinyl-oxy non substitué ou mono- ou disubstitué par A, F, CL, Br, I, OH, OA, OAc, NO₂, NH₂, AcNH, HOOC et/ou AOOC,
R⁶ et R⁷ représentent H, A, HO, AO, CHO, ACO, ACS, HOOC, AOOC, AO-CS, ACOO, A-CS-O, un hydroxyalkylène, un mercaptoalkylène, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkylène, nitroalkylène, cyanoalkylène, A-C(=NOH) ou A-C(=NNH₂),
A un alkyle contenant 1 à 6 atomes de C,
-alkylène un alkylène contenant 1 à 6 atomes de C et
Ac un alcanoyle contenant 1 à 8 atomes de C ou un aroyle contenant 7 à 11 atomes de C,
ainsi que de leurs sels, caractérisé en ce que l'on fait réagir un 3,4-époxychromane de formule II 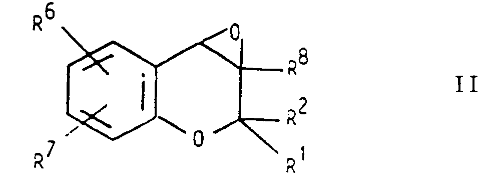 où
R¹, R², R⁶, R⁷ et R⁸ ont la signification indiquée pour la formule I,
avec un composé de formule III
R⁵-H III
où R⁵ a la signification indiquée pour la formule I ou avec l'un de ses dérivés réactifs,
et/ou en ce que l'on déshydrate un composé de formule I où R représente OH et R⁴ H et/ou en ce que l'on transforme dans un composé de formule I un ou plusieurs des restes R³, R⁵, R⁶ et/ou R⁷ en d'autres restes R³, R⁵, R⁶ et/ou R⁷ et/ou en ce que l'on transforme un composé basique de formule I par traitement avec un acide en l'un de ses sels d'addition d'acide.

2. Procédé pour la préparation de compositions pharmaceutiques, caractérisé en ce que l'on met sous une forme de dosage appropriée un composé de formule I selon la revendication 1 et/ou l'un de ses sels physiologiquement acceptables, associé à au moins un support ou un adjuvant solide, liquide ou semi-liquide et éventuellement en combinaison avec une ou plusieurs autre(s) substance(s) active(s).
